# EUROPEAN PATENT APPLICATION

(11) **EP 1 994 901 A1**
(43) Date of publication of application: **26.11.2008**
(21) Application number: 07425317.0
(22) Date of filing: 24.05.2007
(51) Int. Cl.: A61B 17/70

(54) **Intervertebral support device**

(71) Applicant: Bio Medical S.r.L., 50064 Incisa in Val d'Arno, (Firenze) (IT)
(72) Inventor: Mangialardi, Raffaele, 70026 Modugno (Bari) (IT); Piazzolla, Andrea, 70010 Capurso (Bari) (IT); Antonacci, Alberto, 70126 Bari (IT); Carpineti, Giorgio, 6965 Cadro (IT); Perego, Alessandro, 20050 Villa Raverio (Milano) (IT); Sgier, Friederich, 6006 Luzern (IT)
(74) Representative: Lunati, Vittoriano

(57) **Abstract**

An intervertebral support device (1) is provided suitable for being fixed to two spinous processes (102) of two consecutive vertebrae (101) of a spinal column (100) and comprising: two coupling elements (2) suitable for fixing the device (1) to the spinous processes (102), one elastic element (3) in a vertical direction (1b) and connected to the coupling elements (2) and consisting of a folded sheet having a main development plane substantially parallel to the sagittal plane (1d) of the support device (1).

## Description

The present invention concerns an intervertebral support device of the type specified in the preamble of the first claim.

Intervertebral support devices are currently known, in particular devices for intervertebral assisted motion known under the acronym DIAM.

Said support devices are a recent technology for the treatment of problems and pathologies of the spinal column without locally immobilising the same or performing other more complex surgical operations.

They are used to treat above all degeneration of the spinal column and in particular discomfort caused by degeneration of the intervertebral discs. This degeneration, due to age and mechanical stress, consists in a thinning of the intervertebral discs which space and sustain the vertebrae. Said condition causes pain and stiffness of the back.

The intervertebral support devices substantially consist of an elastic element which is positioned between two vertebrae and is suitable for absorbing, in place of the intervertebral disc, the stress and movements that occur between said vertebrae.

In particular said intervertebral support devices are positioned at the level of the spinous processes of the vertebrae, consisting of the rear protuberances of said vertebrae, therefore at a distance from the intervertebral discs.

They are made of elastic materials consisting preferably of silicone, other polymers, metallic materials, such as titanium or stainless steel in particular, or any other biocompatible elastic material.

The known technique cited above has some important drawbacks.

Said intervertebral support devices must have a conformation and physical-mechanical characteristics that adapt to the human body, and in particular to the area between two spinous processes of two adjacent vertebrae, and must have properties that respect the sagittal symmetry of the human body in order to avoid dissymmetry between the right and left side of the human body.

Said devices therefore have dimensions and characteristics subject to specific constraints in terms of dimensions and material, dictated by the necessary biocompatibility and non-corrosion over time inside the human body. Consequently it is difficult to develop intervertebral support devices with the desired elasticity and the necessary resistance to stress and fatigue.

The drawbacks of the intervertebral support devices are the following: an inevitably inappropriate choice in terms of elasticity of the device, reduced resistance to fatigue and reduced resistance of said devices to stress.

In this situation the technical purpose of the present invention is to develop an intervertebral support device able to substantially overcome the above drawbacks.

Within said technical aim, an important object of the invention is to obtain an intervertebral support device having an elasticity that can be substantially chosen as required.

A further object of the invention is to produce an intervertebral support device having a very high resistance to fatigue.

A further important object of the invention is to obtain an intervertebral support device having a correct resistance to stress.

The technical aim and the objects specified are achieved by an intervertebral support device according to the attached Claim 1.

Preferred embodiments are highlighted in the dependent claims.

Further features and advantages of the invention are better explained below in the detailed description of preferred embodiments of the invention, with reference to the attached drawings, wherein:
**Fig. 1** shows an axonometric view of the device according to the invention;
**Fig. 2** illustrates an example of application of the device according to the invention on two vertebrae of a spinal column;
**Fig. 3a** schematises a first lateral view example of the device according to the invention;
**Fig. 3b** schematises a second lateral view example of the device according to the invention;
**Fig. 3c** schematises a third lateral view example of the device according to the invention;
**Fig. 3d** schematises a fourth lateral view example of the device according to the invention;
**Fig. 3e** schematises a fifth lateral view example of the device according to the invention;
**Fig. 3f** schematises a sixth lateral view example of the device according to the invention; and
**Fig. 4** presents an alternative embodiment of the device according to the invention.

With reference to the Figures cited, the intervertebral support device according to the invention is indicated overall by number **1.**

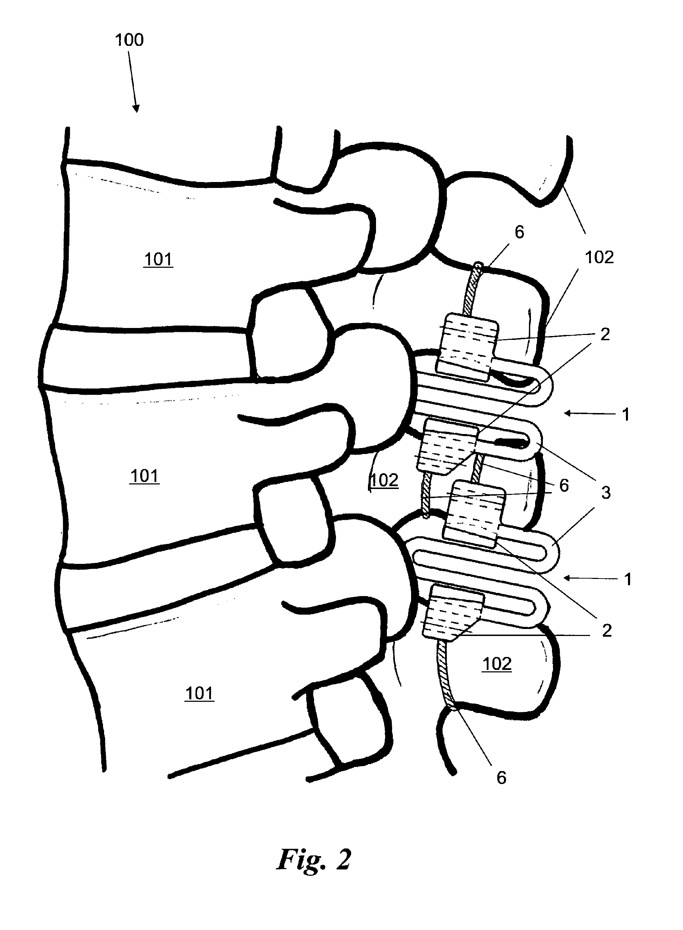

It is suitable for being fixed to two spinous processes **102** of two consecutive vertebrae **101** of the spinal column **100** of the human body or an animal body, as illustrated in Fig. 2, and in particular to two reciprocally facing surfaces of two spinous processes 102, or the upper surface of the lower spinous process 102 and the lower surface of the upper spinous process 102.

According to convention, this patent will use the classic denominations of the planes and axes perpendicular to one another that identify the various directions and symmetries of the human body. In particular the patent will refer to sagittal, frontal and transverse or horizontal planes, and vertical or sagittal, longitudinal and transverse axes.

Consequently, also the support device 1 will comprise guide planes and axes which will be named like the planes and axes that identify the various directions and symmetries of the human body and which, when said support device 1 is fixed inside the human body, coincide substantially with the same planes and axes of the human body.

In particular the longitudinal **1a,** vertical **1b** and transverse **1c** directions of the device 1 and a sagittal plane **1d** of said device 1 are identified.

The support device 1 comprises, summarily, two coupling elements **2** suitable for fixing said device 1 to two spinous processes 102 and at least one elastic element **3,** connected to said support elements and having elastic properties, in particular in the vertical direction 1 b.

In particular two coupling elements 2 are provided arranged in specular positions with respect to the transverse or horizontal plane; the outer surfaces of said coupling elements have a reciprocal distance, once implanted in the spinal column 100, identical to the reciprocal distance which the two facing surfaces of two adjacent spinous processes 102 should have.

More specifically, each coupling element 2 comprises a central recess **4** suitable for being positioned in contact with the spinous process 102 and two lateral flanges **5,** surrounding the central recess and which can be positioned at the sides of the spinous process 102.

Said central recess 4 has an appropriate concave shape with a radius of curvature that is roughly the same as the radius of curvature of the spinous process 102.

The lateral flanges 5 have a roughly parallelepiped form. They have various functions: firstly they maintain the device 1 in a fixed position with respect to the spinous processes 102, and secondly they provide a support for the surgeon during the implant operation.

In order to fix the device 1 to the spinous processes 102, the lateral flanges 5 are positioned at a suitable distance from the central recess 4, so that they can fit onto said spinous processes 102.

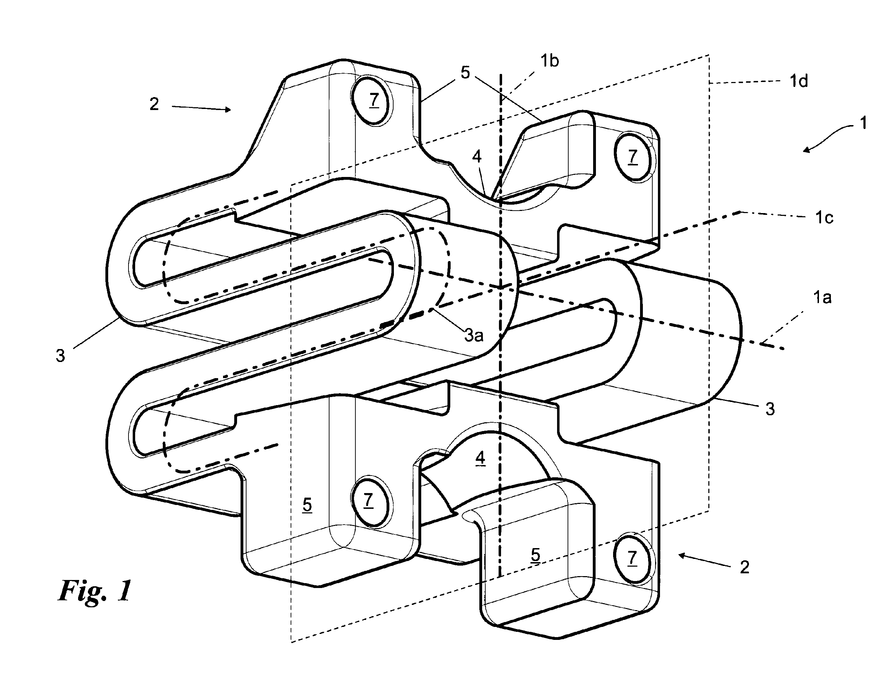

Said lateral flanges have two inner surfaces, facing towards the spinous process 102, which are reciprocally spaced, by means of a chamfer **5a,** at the end that will face towards the axis of the spinal column 100, so as to follow the shape of said spinous process 102, as illustrated in Fig. 1.

Lastly, again in order to follow the shape of the spinous process 102, the coupling element that will be positioned on the upper surface of the lower spinous process 102 has a trapezoidal lateral section, while the coupling element that will be positioned on the lower surface of the upper spinous process 102 has a rectangular lateral section, as illustrated in particular in Fig. 2.

Furthermore, said lateral flanges 5 appropriately comprise connection means **6** of the device 1 to the spinous processes 102.

Said connection means 6 can consist of a ligature (Fig. 2), which envelops the entire spinous process 102 passing through two holes or similar provided on both the lateral flanges 5, or nails or other.

In order to provide the surgeon with a support during implantation, the lateral flanges 5 each include a through hole 7 in a transverse direction, which can be gripped in a stable manner by means of pliers or specific surgical instruments.

The elastic element 3 consists of a folded band-shaped sheet having a main plane of development substantially parallel to the sagittal plane 1d, or in a plane parallel to the vertical direction 1 b and transverse direction 1 c.

In particular two elastic elements 3 are present, arranged symmetrically with respect to the sagittal plane 1d of the support device 1.

Said band is therefore arranged so that its development axis **3a** lies substantially on a plane parallel to the sagittal plane 1 d, while any normal section thereof has a width, in the longitudinal direction 1a, appropriately superior to the height, in the vertical direction 1b.

The elastic element 3 is directly connected to the support elements 2.

Said elastic element 3 can be longitudinally positioned between the coupling elements 2 and in particular between the lateral flanges of two different coupling elements, so that the width in the longitudinal direction 1a of the device 1 does not exceed the width in the longitudinal direction 1 a of the coupling elements 2, or beside said coupling elements 2, so that the width in the longitudinal direction 1a of the device 1 exceeds the width in the longitudinal direction 1 a of the coupling elements 2.

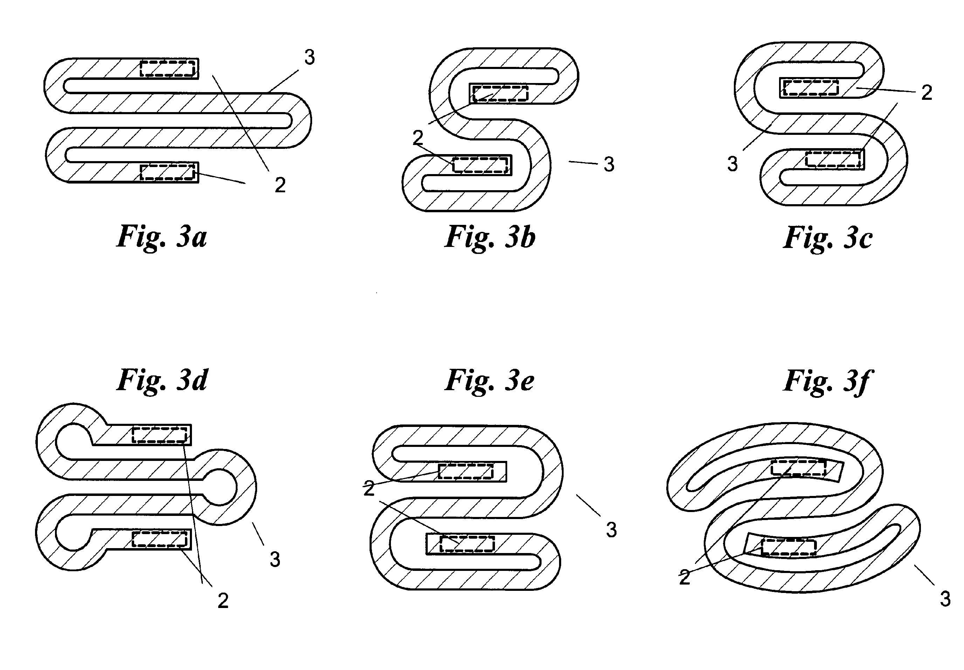

The trajectory of the development axis 3a, and therefore the lateral view of the elastic element 3, can have different paths. Some examples are illustrated in Fig. 3a-3f. In said figures the points of contact with the coupling elements 2 are illustrated by a broken-line rectangle.

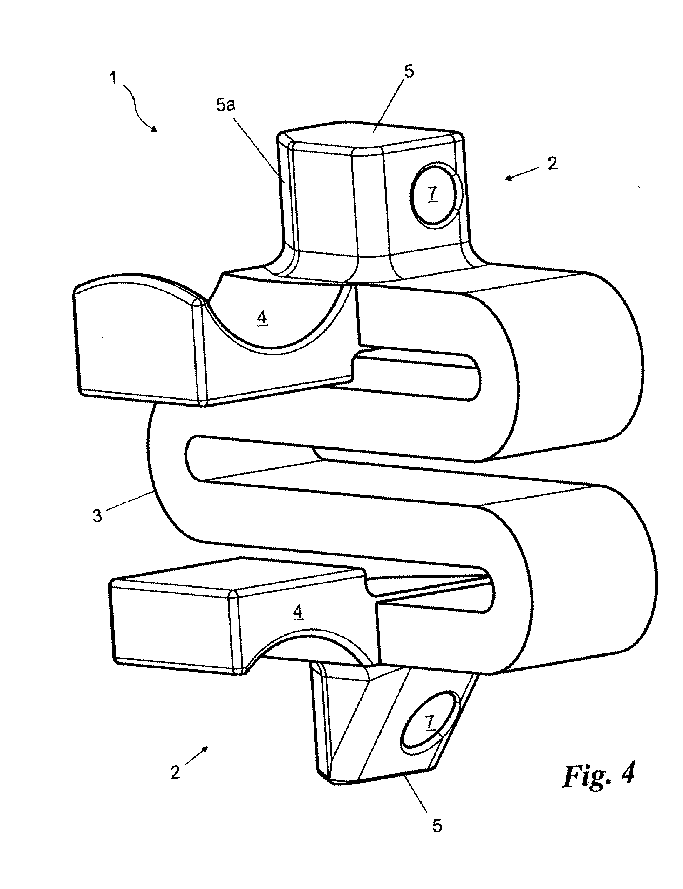

In particular Fig. 1a illustrates the trajectory of the element illustrated in Fig. 1 and 4.

The trajectories illustrated can all be achieved due to the particular arrangement and conformation of the elastic elements 3.

The innovative solution of arranging the elastic elements 3 on a plane parallel to the sagittal plane 1 d means that the same can develop more freely and are not conditioned by the narrowness of said device 1, which is conditioned by the width of the spinous processes 102.

In particular, with this solution the following can be chosen substantially independently: the elastic coefficient of the elastic elements 3, the normal section of the band that forms said elastic elements 3, the curvature of the development axis 3a and also partly the component material.

Due to said freedom of choice and conformation of the elastic elements 3, it is possible to considerably improve the resistance to fatigue and stress of the elastic elements 3 while maintaining their elastic coefficient, which is dictated by the physical needs of the patient.

To improve the resistance to fatigue of the device 1, for example, the elastic elements 3 must be as symmetrical as possible, have broad regular curvature and broad normal section area.

Furthermore, to vary the elastic coefficient of the elastic element 3, it is necessary to vary its total extension in the transverse direction 1 c, given by the sum of the extensions in the same direction of the various planes on which the band-shaped sheet is folded, or vary the normal section of said element 3. In particular an increase in the total extension of the elastic element 3 in the transverse direction 1c causes reduction in the elastic coefficient, while an increase in the area of the normal section, and in particular of the height, of said band-shaped sheet causes an increase in said elastic coefficient.

Consequently, to improve the resistance to fatigue of the elastic element 3 and maintain the correct elastic coefficient it is necessary for said elastic element 3 to have: a large total extension in the transverse direction 1 c, a large area, and in particular height, of the normal section, a regular symmetry and broad regular curvatures.

All the present solutions can be achieved on the support device 1 described. In a different arrangement, according to the known technique, the elastic elements are positioned in the frontal plane rather than in the sagittal plane 1 d. In said devices it is therefore not possible to increase the extension of the elastic elements, thus increasing their elastic coefficient, because the same would reciprocally interfere at the level of the sagittal plane or would interfere with the spatial limits given by the spinal column 100 or directly by the human body.

Said conformation freedom of the elastic elements 3 of the device according to the invention is furthermore considerably improved if the same are positioned beside the support elements 2. In said case, the elastic elements 3 can be vertically positioned outside the coupling elements 2, so that the height in the vertical direction 1b of the device 1 exceeds the height in the vertical direction 1b given by the distance and height of the coupling elements 2 (Fig. 3b, 3c, 3e, 3f).

Lastly, the device 1 is appropriately made of one single material, with the exclusion of the connection means 6, preferably consisting of an alloy of titanium, another biocompatible metal or composite materials such as, in particular, fibre-reinforced materials.

The operation of a support device 1, described above, in structural terms, is the following.

The surgeon inserts said device 1 between two spinous processes 102 and engages it with the same by means of the connection means 6, or simply the coupling elements 2.

To insert the device 1, the surgeon must momentarily cut the supraspinous ligament.

Alternatively, if the device 1 comprises one single elastic element 3 (Fig. 4) it is possible to insert said device laterally without having to cut the supraspinous ligament.

In this case the elastic element 3 should be made of a material with a symmetrical mechanical reaction to compression and traction stress, so that said device 1 maintains a physical-mechanical symmetry with respect to the sagittal plane 1d.

In order to avoid cutting the supraspinous ligament, the device 1 can be divided into two portions, each of which includes an elastic element 3, and can comprise joining means, consisting of pins or similar, suitable for reciprocally joining the two portions. It is preferably divided along the sagittal plane 1d, or along a plane parallel to the same. In particular the two coupling elements 2 are also divided into two portions.

In this latter case, the two halves are appropriately assembled during insertion of the device between the two spinous processes 102.

Once inserted between two spinous processes 102, the support device 1 withstands the stress between the two vertebrae 101 in place of the intervertebral disc, thus solving various types of patient problems, and in particular the problems referred to previously, such as degeneration of the spinal column and in particular degenerative discomfort of the intervertebral discs.

The invention offers important advantages.

The support device 1 can be produced with an appropriate elastic coefficient.

At the same time the device 1 has a high resistance to fatigue and a high resistance to stress.

Furthermore, said device 1 can be shaped according to the physical and structural requirements of the patient, due to the described freedom in terms of production of the same.

## Claims

**1.** Intervertebral support device (1) suitable for being fixed to two spinous processes (102) of two consecutive vertebrae (101) of a spinal column (100) developing mainly in a vertical direction (1b) and being divided into two portions by the sagittal plane (1d), said support device (1) comprising: two coupling elements (2) suitable for fixing said device (1) to said spinous processes (102), at least one elastic element (3) in the vertical direction (1b) and connected to said coupling elements (2) and being **characterised in that** said elastic element (3) consists of a folded sheet having a main plane of development substantially parallel to said sagittal plane (1d) of said support device (1).

**2.** Device according to claim 1, comprising two of said elastic elements (3) arranged symmetrically with respect to said sagittal plane (1d) of said support device (1).

**3.** Device according to one or more of the preceding claims, wherein each of said coupling elements (2) comprises a central recess (4), suitable for being positioned in contact with one of said spinous processes (102), and two lateral flanges (5), positioned at the sides of said central recess (4) and positionable at the sides of said spinous process (102).

**5.** Device according to claim 3, wherein said elastic element (3) is longitudinally positioned between two of said lateral flanges (5) forming part of two different elements of said coupling elements (2).

**6.** Device according to claim 3, wherein said elastic element (3) is longitudinally positioned beside said coupling elements (2).

**7.** Device according to one or more of the preceding claims, wherein said elastic elements (3) extend vertically outside said coupling elements (2).

**8.** Device according to one or more of the preceding claims, comprising only one of said elastic elements (3) positioned laterally with respect to said sagittal plane (1 d) of said support device (1).

**9.** Device according to one or more of the preceding claims, **characterised in that** it is divided into two portions, each including an elastic element (3), and comprises joining means suitable for reciprocally joining said portions.
